# EUROPEAN PATENT APPLICATION

(11) **EP 1 481 690 A1**
(43) Date of publication of application: **01.12.2004**
(21) Application number: 04252933.9
(22) Date of filing: 18.05.2004
(51) Int. Cl.: A61K 47/48

(54) **Therapeutic compositions including bio-availability enhancers**

(30) Priority: 30.05.2003 US 474663 P
(71) Applicant: ROHM AND HAAS COMPANY, Philadelphia, Pennsylvania 19106-2399 (US)
(72) Inventor: Hughes, Lyn, Harleysville Pennsylvania 19438 (US)
(74) Representative: Kent, Venetia Katherine

(57) **Abstract**

The present invention relates to a resin comprising a bio-enhancer and an optional therapeutically active ingredient or precursor thereof loaded thereon. The bio-enhancer is ionizable or non-ionizable, and the resin may be a cation exchange resin or an anion exchange resin.

## Description

### BACKGROUND OF THE INVENTION

The present invention relates to therapeutic compositions that include bio-availability enhancers, and more particularly bio-enhancers and bio-enhancers/therapeutics loaded onto ion exchange resins.

Pharmaceutical formulations are comprised of two classes of ingredient. The first is the active pharmaceutical ingredient (the 'drug') that elicits the desired therapeutic response in the patient. The second class of ingredients are known as excipients that are included to ensure stability, accuracy and precision of dosage form, to improve manufacturing properties, to provide desired therapeutic performance, and as means to controlled release of the active ingredient. These bio-enhancers function in a number of ways, including increasing the solubility of the active ingredient, or increasing the permeability of the active ingredient. On drawback of the art as described in US Provisional Patent Application No. WO2002064543 A2 is that the fumarate salt of O-desmethyl-venlafaxine has unsuitable physicochemical and permeability characteristics.

There have been many attempts to achieve controlled, extended, or modified release of active ingredients. Excipients that are not bio-enhancers have no need for modified or controlled release, indeed they are themselves, used to create modified or controlled release. This is not the case for bio-enhancers. The benefits of controlled or modified release of bio-enhancers can include improved efficacy of the bio-enhancer by delivering it to the optimum site or delivering it at an optimum rate. These benefits can also include protection from adverse conditions that might destroy the bio-enhancer before it reaches its site of optimum activity. An example of this type of adverse condition is the strongly acidic conditions in the stomach which can degrade acid-sensitive compounds.

One of the methods that has been used in the pharmaceutical art to achieve controlled and modified release of drugs is to convert the drug into a complex with an ion exchange resin to form a resinate. Resinates are salts formed between ion exchange resins and ionizable active ingredients. Cation exchange resins form resinates with basic active ingredients. Anion exchange resins form resinates with acidic active ingredients. In the resinate the active ingredient and the resin are in their ionized forms. When resinates are exposed to fluids such as physiological fluids the active ingredient can be released from the resinate by the mechanism of ion exchange. The rate of release of active ingredients from resinates depends on several factors. These include, but are not limited to, the degree of cross-linking of the ion exchange resin, the particle size of the resinate, the pK of the functional groups of the resin, the solubility of the active ingredient in the release fluid, the ionic strength and pH of the release fluid, the pK of the active ingredient, the molecular weight of the active ingredient, and the temperature. Coating the resin with a permeable membrane can also change the rate of release. Coating the resin with non-permeable membranes can change the conditions under which the release takes place depending on the conditions under which the membrane dissolves.

Problems in the art relate to the degradation of bio-enhancers in various hostile media including gastric juices, and/or the controlled delivery of bio-enhancers. None of the art, alone or in combination, teaches or suggests the use of resinates of bio-enhancers to solve bio-enhancer degradation, or solve the problems of therapeutics that have unsuitable physicochemical and permeability characteristics.

### STATEMENT OF THE INVENTION

The present invention relates to resinates of bio-enhancers that can be used to control the delivery of the bio-enhancers. Moreover, a therapeutically active ingredient and an appropriate bio-enhancer can be incorporated into the same resinate. In one variant of the invention, a resin comprising a therapeutically active ingredient and a bio-enhancer loaded thereon is provided.

In another variant of the invention, a resin comprising a bio-enhancer is provided. The bio-enhancer is ionizable or non-ionizable. In yet other versions the resin is a cationic resin or an anionic resin. In yet further variants, the anionic resin is a styrenic strongly basic anion exchange resin. In various embodiments, the styrenic strongly basic anion exchange resin further comprises quaternary amine functionality or tertiary amine functionality.

In yet other aspects, the resin is a styrenic strongly acidic cation exchange resin with optional sulfonic functionality, phosphonic acid functionally, or a combination thereof. In yet a further variant, the resin is a cation exchange resin having carboxylic functionality, phenolic acid functionally, or a combination thereof.

These and other variants of the invention are provided in the specification herein.

### DETAILED DESCRIPTION OF THE INVENTION

The following terms used herein, in addition to having the meanings known in the art, have the following meanings herein:
The term 'bio-enhancer' means a substance included in a pharmaceutical dosage form that serves to increase the bio-availability of the active ingredient to a subject *in vivo.*
The term "release rate profile", as used herein, means the rate at which the active ingredient that is loaded on the resin appears in solution in the release medium. This can be expressed in terms of the instantaneous concentration of the active ingredient in solution as a function of time, or expressed in terms of the percentage of total active ingredient available that has appeared in solution in the release medium as a function of time.
The term "release medium" and as used herein, means the liquid medium into which the active ingredients is being released. Examples of release media can be water, simulated intestinal fluid, simulated gastric fluid, simulated saliva, or the authentic physiological versions of these fluids, water, and various buffer solutions.
The term "ion exchange resin", as used herein, means any insoluble polymer that can act as an ion exchanger.
The term "release", as used herein, means the transfer of active ingredient from the resinate into the release medium. When applied to a resin or resinate, the term "absorption", as used herein, means the reverse of release, namely the transfer of active ingredient from the medium into the ion exchange resin or resinate.
The term "water retention capacity" as used herein is used to describe the maximum amount of water that an ion exchange resin can retain within the polymer phase and in any pores. (ASTM D2187: Standard Test Methods for Physical and Chemical Properties of Particulate Ion Exchange Resin. Test Method B: Water Retention Capacity)
The term "resinate," means a complex formed between an active ingredient, a bio-enhancer, or active ingredient and a bio-enhancer, and an ion exchange resin. It is also known as a loaded resin. The term "resinate" can also relate to an active ingredient/ion exchange resin complex, an active ingredient/bio-enhancer resin complex, or a bio-enhancer resin complex.

Further, ion exchange resins are characterized by their capacity to exchange ions. This is expressed as the "Ion Exchange Capacity." For cation exchange resins the term used is "Cation Exchange Capacity," and for anion exchange resins the term used is "Anion Exchange Capacity." The ion exchange capacity is measured as the number equivalents of an ion that can be exchanged and can be expressed with reference to the mass of the polymer ( herein abbreviated to "Weight Capacity") or its volume (often abbreviated to "Volume Capacity"). A frequently used unit for weight capacity is "milliequivalents of exchange capacity per gram of dry polymer." This is commonly abbreviated to "meq/g."

Ion exchange resins are manufactured in different forms. These forms can include, by way of example, spherical and non-spherical particles with size in the range of 0.00001mm to 2mm. The non-spherical particles are frequently manufactured by grinding of the spherical particles. Products made in this way typically have particle size in the range 0.0001mm to 0.2mm. The spherical particles are frequently known in the art as 'Whole Bead.' The non-spherical particles are frequently known in the art as 'Powders.'

Resin/therapeutically active ingredient complexes can be prepared by any of the methods know in art. Compositions of the current invention can include the active ingredient in pure form or as a resinate. In cases where the active ingredient and the bio-enhancer are both acidic, or both basic, or both non-ionizable, the active ingredient and the bio-enhancer can be loaded onto the same resin. For example, if the drug has a carboxylic acid group it can be loaded onto an anion exchange resin. If the bio-enhancer has a sulfonic acid or carboxylic acid it can be loaded onto the same anion exchange resin. The resulting resinate contains both the active ingredient and the bio-enhancer.

Compositions of the current invention include the active ingredient in pure form or as a resinate. In cases where the active ingredient and the bio-enhancer are both acidic, or both basic, or both non-ionizable, the active ingredient and the bio-enhancer can be loaded onto the same resin. For example, if the drug has a carboxylic acid group it can be loaded onto an anion exchange resin. If the bio-enhancer has a sulfonic acid or carboxylic acid it can be loaded onto the same anion exchange resin. The resulting resinate contains both the active ingredient and the bio-enhancer.

Ion exchange resins useful in the practice of the present invention include, but are not limited to, anionic exchange resins and cationic exchange resins. Preferably, the resins are suitable for human and animal ingestion when the application is pharmaceutical. The following are examples of ion exchange resins that are used in variants of the invention: anionic exchange resins include, but are not limited to, styrenic strongly basic anion exchange resins with a quaternary amine functionality having a weight capacity of 0.1 to 15 meq/g, and styrenic weakly basic anion exchange resins with a primary, secondary, or tertiary amine functionality having a weight capacity of 0.1 to 8.5 meq/g, and acrylic or methacrylic strongly basic anion exchange resins with a quaternary amine functionality having a weight capacity of 0.1 to 12 meq/g, and acrylic or methacrylic weakly basic anion exchange resins with a primary, secondary, or tertiary amine functionality having a weight capacity of 0.1 to 12 meq/g, and allylic and vinylic weakly basic anion exchange resins with a primary, secondary, or tertiary amine functionality having a weight capacity of 0.1 to 24 meq/g.

In another variant of the invention, anionic exchange resins include, but are mot limited to, styrenic strongly basic anion exchange resins with a quaternary amine functionality having a weight capacity of 0.1 to 6 meq/g, and styrenic weakly basic anion exchange resins with a tertiary amine functionality having a weight capacity of 0.1 to 8.5 meq/g, acrylic or methacrylic strongly basic anion exchange resins with a quaternary amine functionality having a weight capacity of 0.1 to 8 meq/g, and acrylic or methacrylic weakly basic anion exchange resins with a tertiary amine functionality having a weight capacity of 0.1 to 12 meq/g, and allylic and vinylic weakly basic anion exchange resins with primary, secondary, or tertiary amine functionalities having a weight capacity of 0.1 to 24 meq/g.

In yet other variants of the invention, anionic exchange resins include, but are not limited to, styrenic strongly basic anion exchange resins with quaternary amine functionality with weight capacity of 0.1 to 6 meq/g and acrylic anion exchange resins with tertiary amine functionality with weight capacity of 0.1 to 12 meq/g. Styrenic strongly basic anion exchange resins with quaternary amine functionalities with weight capacities of 4.0 to 4.5 meq/g are also known as cholestyramine resins.

In yet other variants of the invention cationic exchange resins include, but are not limited to, styrenic strongly acidic cation exchange resins with sulfonic or phosphonic acid functionalities having a weight capacity of 0.1 to 8 meq/g; and styrenic weakly acidic cation exchange resins with carboxylic or phenolic acid functionalities having a weight capacity of 0.1 to 8.5 meq/g; and acrylic or methacrylic weakly acidic cation exchange resins with a carboxylic or phenolic acid functionality with a weight capacity of 0.1 to 14 meq/g.

In yet further variants of the invention cationic exchange resins include, but are not limited to, styrenic strongly acidic cation exchange resins with a sulfonic acid functionality having a weight capacity of 0.1 to 8 meq/g; and styrenic weakly acidic cation exchange resins with a phenolic acid functionality having a weight capacity of 0.1 to 8.5 meq/g; and acrylic or methacrylic weakly acidic cation exchange resins with a carboxylic or phenolic acid functionality with a weight capacity of 0.1 to 14 meq/g.

In yet other variants of the invention, cationic exchange resins include, but are not limited to, styrenic strongly acidic cation exchange resin with sulfonic acid functionality with weight capacity of 0.1 to 8 meq/g, and acrylic or methacrylic weakly acidic cation exchange resin with a carboxylic acid functionality with weight capacity of 0.1 to 14 meq/g.

Ion exchange resins useful in this invention, in one variant, have a moisture content between 0% and the water retention capacity of the resin. Ion exchange resins useful in this invention are in powder or whole bead form. Strongly acidic and weakly acidic cation exchange resins useful in the practice of the present invention are in the acid form or salt form or partial salt form in yet further variants of the invention. Strongly basic anion exchange resins useful in this invention are in the salt form in variants of the invention. Weakly basic anion exchange resins useful in this invention are in the free-base form or salt form or partial salt form in variants of the invention. The particle size of resins useful in the invention will be defined by the desired release rate profile and can be determined empirically by artisans. Typical particle size ranges are from 0.00001mm to 2mm in one variant of the invention. Other size ranges useful in the invention are from 0.001mm to 1mm, and from 0.001mm to 1.0mm

The compositions of the present invention can optionally be coated. Permeable coatings useful in this invention include Eudragit® RL100, and Eudragit® RS100 (Rohm-Pharma Darmstadt, Germany). Non-permeable coatings useful in this invention include Aquacoat® CPD (FMC Corporation, Philadelphia, PA, USA), Eudragit® E100, Eudragit® L100, Eudragit® S100 (Rohm-Pharma Darmstadt, Germany), Kollicoat® MA 30 DP (BASF Aktiengesellschaft, Ludwigshafen, Germany).

Active ingredients useful in the practice of the present invention include, but are not limited to, physiologically active ingredient and vitamins. Other active ingredients include: antimicrobial agents such as: ciprofloxacin, cefmatazole, perfloxacin, ofloxacin, norfloxacin, phenoxymethyl penicillin, ampicillin, amoxycillin, erythromycin, cloxacillin, roxithromycin, azithromycin, cephalexin, cefadroxil, cerfuoxime, cerfuoxime axetil, cefixime, cotrimoxazole, acyclovir, cefaclor, clofazimin, fluconazole, griseofulvin, ketoconazole; antiprotozoal agents such as: metronidazole, tinidiazole, quinine, chloroquine, primaquine, sulfadoxine, pyrimethamine; anthelmintic agents such as mebendazole, praziquantel, pyrantel, febantel; cardiovascular drugs such as: amlodipine, diltiazem, atenolol, lisinopril, lovastatin, gemfibrozil, nifedipine, enalapril, propanolol; drugs acting on the central nervous system: L-dopa, buspirone, dextropropoxyphene, pentazocine, morphine derivatives, diazepam, lorazepam, alprazolam, haloperidol, chlorpromazine, sulpiride, thioridazine, venlafaxine, O-desmethoxyvenlafaxine; non-steroidal anti-inflammatory drugs such as: diclofenac, ketorolac, piroxicam, ibuprofen, indomethacin, naproxen; drugs used in treatment of respiratory disorders such as: solbutamol, terbutaline, theophylline, bromhexine;a ntihistaminics such as: astemizole, terfenidine, loratidine; prokinetic drugs such as: metoclopramide, domperidone, cisapride; corticosteroids such as: prednisolone, dexamethasone, betamethasone; steriod hormones such as: stanazolol, oral contraceptives; vitamins such as A, C, E, and K; antiulcer drugs such as: omerazole, ranitidine, femotidine; central muscle relaxants such as: carisoprodol, chlormezanone; anticancer drugs such as the alkylated agents: merchlorthiamine, cyclophosphamide, ifosamide, chlorambucil, hexamethylmelamine, thiotepa, busulfan, carmustine, lomustine, semustine, streptozotocin, decarbazine; anticancer drugs such as the anitmetabolites: methatrexate, 5-flurouracil, floxuridine, cytosine arabinoside, 6-mercaptopurine, thioguanine, pentostatin; anti-cancer drugs that are natural products such as: vincristine, vinblastin, etoposide, dectinmycin, daunorubicin, doxorubicin, epirubicin, idarubicin, bleomycin, methramicin, mitomycin, L-asparaginase; anti-cancer drugs such as: cisplatin, carboplatin, mitoxantrone, hydroxyurea, procarbazine, mitotane, aminoglutathimide; hormones and hormone antagonists such as: prednisilone, hydroxyprogestirone, medroxyprogestirone, megestrol, diethyl stilbestrole, ethinyl estradiol, tamoxifen, testosterone, fluoxymestrone, flutamide, leuprolide. It is appreciated that precursors and derivatives of the above captioned drugs can also be used in the present invention.

The active ingredient component of the composition may be present in any amount which is sufficient to elicit a beneficial effect.

The bio-enhancer component of the composition may be present in any amount which is sufficient to elicit a beneficial effect. Examples of bio-availability enhancers include: monocarboxylic acids and salts thereof, polycarboxylic acids and salts thereof, sulfonic acids and salts thereof, amines and salts thereof, saponins, mono-, di-, and triglycerides. In other variants, examples of bio-enhancers include, but are not limited to ascorbic acid, citric acid, malic acid, fumaric acid, succinic caproic acid, caprylic acid, glycocholic acid, cholic acid, 2-n-octyldodecanoic acid, lauryl hydrogen sulfate, lipoic acid, palmitoyl carnitine, amino acids, piperine, tetrahydropiperine, 4-Acridinecarboxamide, N-[4-[2-(3,4-dihydro-6,7-dimethoxy-2(1H)-isoquinolinyl)ethyl]phenyl]-9,10-dihydro-5-methoxy-9-oxo- (9CI), also known as Elacridar, tromethamine, di-octyl dosium sulfosuccinate, deoxychlate, glycocholate, dodecylmaltoside, oleic acid, linoleic acid, linolenic acid, N-acetylated amino acids, lauryl carnitine, mono-, di-, and triglycerides of caproic acid, mono-, di-, and triglycerides of caprylic acid,acyl cholines, alkanoyl cholines

The final dosage form can be any of the many variations known in the art. These can include, but are not limited to, creams, tablets, powders, pills, syrups, hard capsules and soft capsules.

The invention is not restricted to the use of only one loaded resin. Multiple, the same type or different type, loaded resins can also be used as needed to produce the desired therapeutic result.

The invention is not restricted to the use of only one active ingredient or bio-enhancer. Multiple active ingredients or bio-enhancers can be used to produce the desired beneficial effect on a single resin or on multiple resins.

Mixtures of resins can also be used in the present invention. By way of example a first resin having a therapeutically active compound thereon is mixed with a second resin having a bio-enhancer thereon. Dosages of the first and second resin are determined empirically. The first resin is then mixed with the second resin and provided in a dosage form to a subject.

In yet a further variant, the invention provides for a method of administering a drug such that the drug has increased bio-availability. The method includes administering the drug with a resin having a bio-availability enhancer in one variant. In another variant, the method includes administering a resin having a bio-availability enhancer and an optional therapeutic thereon.

In one variant, the present invention loads a novel salt of O-desmethyl-venlafaxine, O- desmethyl-venlafaxine succinate, or precursors, or derivatives thereof (hereinafter referred to as "ODV succinate") onto one or more resins of the present invention. In one variant, the succinate salt is loaded on one site of a first resin, and the O-desmethyl-venlafaxine is loaded onto a second site of the same resin. In another variant, the succinate salt is loaded on one site of a first resin (bead or typef), and the O-desmethyl-venlafaxine is loaded onto a site of a second resin (bead or type). The succinate salt acts as the bio-availability enhancer for the therapeutic compound. Such compounds are described in US Provisional Patent Application No. WO2002064543 A2 entitled "NOVEL SUCCINATE SALT OF O-DESMETHYL-VENLAFAXINE."

The salt form using the present invention has properties which are particularly suitable for use as a drug, including improved solubility, permeability, and bioavailability. For example, ODV/succinate resin complex acts such that the active ingredient is well absorbed in the gastrointestinal tract. Furthermore, it is contemplated that oral administration of ODV succinate resin complex may result in a lower incidence of nauseas, vomiting, diarrhea, abdominal pain, headache, vaso-vagal malaise, and/or trismus than oral administration of venlafaxine, O-desmethyl-venlafaxine, and salts of O-desmethyl 10 venlafaxine other than ODV succinate. Additionally, sustained release oral formulations of ODV succinate result in a lower incidence of nauseau, vomiting, diarrhea, abdominal pain, headache, vaso-vagal malaise, and/or trismus than oral administration of venlafaxine, O-desmethyl-venlafaxine, and salts of O-desmethyl- venlafaxine (other than sustained release oral formulations of ODV succinate).

Pharmaceutical compositions comprising ODV/succinate resin complexes and pharmaceutically acceptable carriers or excipients are also provided. Preferably, the pharmaceutical compositions comprise an amount of ODV succinate effective to treat the desired indication in an animal, such as a human.

In further embodiments of the present invention are provided methods of treating patients suffering from depression (include, but not limited to, major depressive disorder, bipolar disorder, and dysthymia), anxiety, panic disorder, generalized anxiety disorder, post traumatic stress disorder, premenstrual dysphoric disorder, fibromyalgia, agoraphobia, attention deficit disorder (with and without 25 hyperactivity), obsessive compulsive disorder (including trichotillomania), social anxiety disorder, autism, schizophrenia, obesity, anorexia nervosa, bulimia nervosa, Gilles de la Tourette Syndrome, vasomotor flushing, cocaine and alcohol addiction, sexual dysfunction (including, but not limited to, premature ejaculation), borderline personality disorder, chronic fatigue syndrome, urinary incontinence, pain (including, 30 but not limited to, migraine, chronic back pain, phantom limb pain, central pain, neuopathic pain such as diabetic neuropathy, and postherpetic neuropathy), Shy Drager syndrome, Raynaud's syndrome, Parkinson's disease, and epilepsy comprising providing to a patient an effective amount of ODV succinate. ODV succinate can also be administered to prevent relapse or recurrence of depression, to -2 induce cognitive enhancement, to treat cognitive impairment, and in regimens for cessation of smoking or other tobacco uses using the present invention. Additionally, ODV/succinate resin complexes or mixtures as described herein can be administered to treat hypothalamic amenorrhea in depressed and non-depressed human females. These methods include administering to a patient in need thereof, an effective amount of the compounds described herein or a substantially pure polymorph of ODV in the compositions described herein, or mixtures thereof.

The present invention uses in one variant a novel salt of O-desmethyl-venlafaxine, O desmethyl-venlafaxine succinate or components thereof, e.g. the succinate salt and the remaining therapuetic (hereinafter referred to as "ODV succinate"). ODV succinate provides optimal properties for formulation due to its high solubility, permeability, and bioevailability. Succinic acid salts of O- desmethyl-venlafaxine exist as enantiomers and this invention optionally uses racemic mixtures as well as stereoisomerically pure forms of the same. The term "ODV succinate" as used herein refers to racemic mixtures and stereoisomerically pure forms of ODV succinate, precursors thereof, and derivatives thereof.

The term "stereoisomerically pure" refers to compounds which are comprised of a greater proportion of the desired isomer than of the optical antipode. A stereoisomerically pure compound is generally made up of at least about 90% of the desired isomer, based upon 100% total weight of ODV succinate.

Succinic acid is a dicarboxylic acid and the invention therefore includes both salts in which the ratio of O-desmethyl-venlafaxine to acid (by mole) is 1:1 (i.e., a monosuccinate) and salts in which the ratio of O- desmethyl-venlafaxine to acid (by mole) is 2:1 (i.e., a his isuccinate), as well as mixed salts, with for example an alkali metal or ammonium cation. The invention also uses mixtures of ODV succinate and the free base of O-desmethyl-venlafaxine. The crystalline polymorphs (i.e. Forms I, II, III, and IV) and the amorphous form of ODV succinate are monosuccinate salts, or components thereof, i.e., the molar ratio of O- desmethyl-venlafaxine to acid is 1:1, are used in one variant of the invention.

Salts of the present invention can be crystalline and may exist as more than one polymorph. Each polymorph forms another aspect of the invention. Hydrates as well as anhydrous forms of the salt are also used in the invention. In one variant, the monobydrate form of O-desmethyl venlafaxine succinate is used. ODV succinate generally has a solubility in water of greater than 30 mg/mL. In another variant, the aqueous solubility of the ODV succinate is at least 25, 30, 32, 35, 40, or 45 mg/mL at 25° C. Succinic acid salts may be formed by contacting stoichiometric amounts of the acid with O-desmethy- venlafaxine free base. Alternatively, the acid may be used in excess, usually no more than 1.5 equivalents.

Different forms of O-desmethyl-venlafaxine can be used in the present invention.

### Example 1 - Active Ingredient Resinate

In this example,e the resin used is a strongly acid cation exchange resin in the hydrogen form with a weight capacity of 4.5meq/g on a dry basis. The moisture content of the resin is 6% w/w. The resin is in a powder form. The active ingredient is sulpiride. 9.2g of resin is mixed with 350ml of 25% ethanol in water. To this is added 3.3g of sulpiride. The mixture is agitated for 12 hours and then filtered. The filtrate is washed with water and then dried to constant weight *in vaccuo* at 60°C. The resinate contains approximately 0.27g of sulpiride per gram of resinate.

### Example 2 - Bio-enhancer resinate

In this example, the resin used is a hydrated strongly basic anion exchange resin in the chloride form with a weight capacity of 4.25meq/g on a dry basis. The moisture content of the resin is 70% w/w. The resin is in a powder form. The bio-enhancer is decanoic acid. 33.3g of resin is mixed with 400ml of water. To this is added 5.4g of sodium decanoate. The mixture is agitated for 12 hours at 50°C and then filtered. The filtrate is washed with water and then dried to constant weight *in vaccuo* at 60°C. The resinate contains the equivalent of approximately 0.17g of sodium decanoate per gram of resinate.

### Example 3- Desmethyl venlafaxine/succinate resinate

This example demonstrates the preparation of a resinate containing both the active ingredient and the bio-enhancer. The resin used is strongly basic anion exchange resin in the hydroxide form with a weight capacity of 4.6meq/g on a dry basis. The moisture content of the resin is 6% w/w. The resin is in a powder form. 5.0g of the resin is mixed with 500ml of 25% ethanol. 1.16g of O-desmethylvenlafaxine are added and the mixture is agitated for 6 hours. 0.53g of succinic acid is then added and the mixture agitated for a further 12 hours. The mixture is then filtered and the filtrate is washed with 25% ethanol and then with water. The washed filtrate is then suspended in 150ml water and 1% hydrochloric acid is added very slowly until the pH of the supernatent is ~10. The mixture is filtered and washed with water. The washed filtrate is dried *in vaccuo* at 60°C. One gram of the resulting resinate contains approximately the equivalent of 0.25g O-desmethylvenlafaxine succinate salt.

## Claims

1. A resin comprising a bio-enhancer and an optional therapeutically active ingredient or precursor thereof loaded thereon.

2. The resin of claim 1 in which said bio-enhancer is ionizable.

3. The resin of claim 1 in which said bio-enhancer is non-ionizable.

4. The resin of claim 1 that is an anion exchange resin.

5. The resin of claim 4 in which said anion exchange resin is a styrenic strongly basic anion exchange resin.

6. The resin of claim 5 in which said styrenic strongly basic anion exchange resin further comprises quaternary amine functionality.

7. The resin of claim 5 in which said resin is an acrylic anion exchange resin with tertiary amine functionality.

8. The resin of claim 1 in which said resin is a cation ion exchange resin.

9. The resin of claim 8 in which said cationic ion exchange resin is a styrenic strongly acidic cation exchange resin.

10. The resin of claim 9 in which said styrenic strongly acidic cation exchange resin further comprises sulfonic functionality, phosphonic acid functionally, or a combination thereof.

11. The resin of claim 9 in which said styrenic cation exchange resin comprises carboxylic functionality, phenolic acid functionally, or a combination thereof.
